# EUROPEAN PATENT APPLICATION

(11) **EP 2 153 844 A1**
(43) Date of publication of application: **17.02.2010**
(21) Application number: 08014518.8
(22) Date of filing: 14.08.2008
(51) Int. Cl.: A61K 38/47, A61P 25/00

(54) **Human hyaluronidases for axonal regrowth**

(71) Applicant: HAUBECK, Hans-Dieter, 55435 Gau-Algesheim (DE)
(72) Inventor: HAUBECK, Hans-Dieter, 55435 Gau-Algesheim (DE)
(74) Representative: Weiss, Wolfgang

(57) **Abstract**

Chondroitin sulfate proteoglycans (CS-PGs) are axon growth inhibitory molecules present in the glial scar that are responsible (at least in part) for regeneration failure after CNS or spinal cord injury. Removal of chondroitin sulfate glycosaminoglycan chains using the bacterial enzymes chondroitinase-ABC or AC in models of CNS injury promotes both axon regeneration and plasticity.

The present invention relates to the use of members of the human hyaluronidase family (endo-beta-acetyl-hexosaminidase enzymes, E.C. 3.2.1.35) for the degradation of chondroitin sulfate (proteoglycans) in the glial scar to promote axonal regrowth in human CNS or spinal cord injury.

The present invention also relates to methods for determining endoglycosidase activity, and in particular of the hyaluronidase/chondroitinase type, in a sample, and also relates to methods for detecting compounds that modulate the activity of endoglycosidases and in particular endoglycosidases of the hyaluronidase/chondroitinase type.

## Description

### FIELD AND STATE OF THE ART

Injury to the central nervous system (CNS) or to the spinal cord (SC) induces tissue damage and profoundly alters nerve function.
Spinal cord injury (SCI) often induces loss of motor and/or sensory function below the level of injury. Depending on the severity of the injury these deficits will persist, which causes enormous strain and distress to patients. Furthermore, treatment of these patients is extremely expensive for public health care systems.
Therefore CNS and SC injury represent a clinical problem of major importance and novel therapeutic approaches are urgently needed.

However, the mechanisms underlying the lack of recovery in the adult CNS (and SC) have been clarified only partially at the molecular level. Knowledge of these mechanisms should therefore provide the basis for the development of new therapeutic strategies for CNSI and SCI.

In the adult CNS, a standard response to injury is the formation of a glial scar, which quickly seals off the injured site from healthy tissue - preventing uncontrolled tissue damage, for example by bacterial invasion. However, glial scarring also blocks the long-term regeneration of damaged axons. Scar tissue is made up of reactive astrocytes, oligodendrocyte precursors, microglia/macrophages, meningeal cells and vascular endothelial cells and contains axon growth inhibitory molecules.

Following injury to the mammalian CNS, those axons that attempt to regenerate face several inhibitory challenges. The result is failure of regeneration or limited local sprouting (Fawcett J W. Adv. Exp. Med. Biol. 557, 11-24, 2006; Rhodes KE, Fawcett JW. J. Anat. 204, 33-48, 2004; Silver J., Miller JH., Nat. Rev. Neurosci. 5: 146-156, 2004).

Increased expression of certain extracellular matrix (ECM) molecules after CNS injury is believed to inhibit/restrict axonal regeneration.

Chondroitin sulfate proteoglycans (CS-PG) are a class of ECM molecules that inhibit neurite outgrowth and are rapidly upregulated after CNS injury (Silver J., Miller JH. Nat. Rev. Neurosci. 5: 146-156, 2004; Busch SA, Silver J. Curr. Opin. Neurobiol. 17, 120-127, 2007; Jones LL, et al. J. Neurosci. 23, 9276-9288, 2003; Lu P, et al. Exp. Neurol. 203, 8-21, 2007). The role of CS-PG in reducing axon growth after injury has been studied extensively (Levine JM, J Neurosci. 14: 4716- 4730,1994; Jones LL, et al. J. Neurosci. 23, 9276-9288, 2003, Ughrin YM, et al., J. Neurosci. 23: 175-186; Tan AM et al., J. Anat. 207: 717-725, 2005) and it has been shown in vitro and in-vivo (in several animal models), that CS-PG, produced mainly by reactive astrocytes and oligodendrocyte precursor cells (OPC) in glial scars, restrict neurite outgrowth (see below [0008] - [0009]).

CS-PG are complex macromolecules of the ECM that are expressed in a wide variety of tissues and cells of human and nonhuman origin. The principal structure of CS-PG consists of a core protein, to which one or several chondroitin (or dermatan) sulfate chains are covalently attached. The linear polysaccharide chains generally consist of repeating disaccharide subunits composed of a glucuronic acid (or in dermatan sulfate alternatively iduronic acid) and galactosamine. These disaccharides are substituted to a varying degree with sulfate groups and N-linked acetyl residues. In the past, chondroitin sulfates have been subdivided into different forms, e.g. chondroitin sulfate A (chondroitin-4-sulfate), chondroitin sulfate C (chondroitin-6-sulfate) and chondroitin sulfate B (dermatan sulfate, that contains the C-5 epimeric iduronic acid instead of glucuronic acid residues) (Hoffman et al. Fed. Proc. 17, 1078-1082, 1958).
However, in the following years a more detailed analysis has shown that chondroitin sulfates may have more heterogenous structures (Suzuki S, et al. J. Biol. Chem. 243 : 1543-1550, 1968).

After CNS injury, the expression and synthesis of several CS-PG is increased and the developing glial scar contains large amounts of CS-PG. The relative increase during post-injury periods is different for these CS-PG, e.g. Neurocan, Brevican, Versican, Phosphacan and NG2. Various lines of evidence demonstrate that much of the axon growth inhibitory activity of CS-PG comes from their glycosaminoglycan (GAG) chains (Levine JM, J Neurosci. 14: 4716-4730,1994; Jones LL, et al. J. Neurosci. 23, 9276-9288, 2003, Ughrin YM, et al., J. Neurosci. 23: 175-186; Tan AM et al., J. Anat. 207: 717-725, 2005).

Direct evidence for the important role of CS-PG in the impaired axonal regeneration after CNS or spinal cord injury (but also peripheral nerve injury) comes from several in-vitro and in-vivo experiments.
In these experiments degradation of chondroitin sulfate glycosaminoglycan chains by the bacterial enzymes chondroitinase ABC or chondroitinase AC (see below [0012-[0013]) can overcome the inhibition of axonal growth by CS-PG and promote nerve regeneration and functional recovery (Groves ML et al., Exp. Neurol. 195: 278-292, 2005; Lu P et al., Exp. Neurol. 203, 8-21, 2007; Bradbury EJ et al., Nature 416, 636-640, 2002; Snow DM et al., Exp. Neurol. 109, 110-130, 1990).

Endoglycosidases are enzymes capable of cleaving polymers comprising glycosaminoglycan chains, for example, chondroitin sulfate glycosaminoglycans.

The catabolism of glycosaminoglycans (GAG) and especially of chondroitin sulfate has been studied in detail in the past (Glaser JH, Conradt HE, J Biol. Chem. 254, 2316-2325, 1979; Ingmar B, Wasteson A., Biochem. J. 179, 7-13, 1979) and the sequential degradation of a chondroitin sulfate trisaccharide by different lysosomal exoglycosidases was shown (Glaser JH, Conradt HE, J Biol. Chem. 254, 2316-2325, 1979). However, a human homologue to the bacterial enzymes chondroitinase ABC or chondroitinase AC (see below [0012] - [0014]) has not been described.

Bacterial enzymes have been isolated and characterized, that degrade chondroitin sulfates (Yamagata T et al., J. Biol. Chem. 243, 1523-1535, 1968; Suzuki S, et al. J. Biol. Chem. 243 : 1543-1550, 1968; Sanderson PN et al., Biochem. J. 257, 347-354, 1989).

According to its specificity for the different chondroitin sulfate types (see above [0007]) the enzyme purified from extracts of *Proteus vulgaris* was given the name **chondroitinase ABC.** A second chondroitinase, **chondroitinase AC,** has been purified from *Flavobacter heparinum* (Yamagata T et al., J. Biol. Chem. 243, 1523-1535, 1968; Suzuki S, et al. J. Biol. Chem. 243 : 1543-1550, 1968).
In contrast to chondroitinase AC, chondroitinase ABC will cleave also dermatan sulfate (chondroitin sulfate B; Sanderson PN et al., Biochem. J. 257, 347-354, 1989).
Both enzymes are endoglycosidases and carry out an elimination reaction, yielding Δ4,5-unsaturated disaccharides (Yamagata T et al., J. Biol. Chem. 243, 1523-1535, 1968).

In the past, the endogylcosidases chondroitinase AC and ABC have been used extensively for structural analysis of glycosaminoglycans and especially of chondroitin sulfate and dermatan sulfate (proteoglycans).

However, the strong upregulation of chondroitin sulfate synthesis after CNS or spinal cord injury and the high content of chondroitin sulfate (proteoglycans) within glial scars have suggested that CS-PG are axon growth inhibitory molecules that play a major role in regeneration failure after damage to the CNS and which restrict CNS plasticity.

Enzymatic removal of the glycosaminoglycan (GAG) side chains by chondroitinase ABC (or chondroitinase AC), has been shown to render CS-PG less inhibitory to axon growth, and to render glial cells more permissive. Chondroitinase ABC therefore can contribute to local degradation of chondroitin sulfate and thus facilitate cell migration and passage of growth cones across the glial scar (Zuo J et al., Exp. Neurol. 154, 654-662, 1998; Smith-Thomas L, et al. J. Cell Sci. 107, 1687-1695, 1994, Smith-Thomas L, et al. J. Cell Sci. 108, 1307-1315, 1995, Curinga GM et al. J. Neurochem. 102, 275-288, 2007). These data show that much of the axon growth inhibitory activity of CS-PG comes from their glycosaminoglycan (GAG) chains.

Application of chondroitinase ABC to mammalian brain and spinal cord injury in vivo (in different animal models), has shown increased regeneration of injured nerve fibres, increased plasticity and recovery of function (Moon et al. Nat. Neurosci. 4, 465 -466, 2001; Bradbury EJ et al., Nature 416, 636-640, 2002; Houle JD et al., J. Neurosci. 26, 7405-7415, 2006).

In most of these studies chondroitinase ABC was infused several times as a bolus (for example on alternate days). The rationale for this regimen was that extracellular matrix turnover in the CNS has been shown to be relative slow. Therefore it may be (probably) sufficient to achieve an enzyme concentration that degrades CS GAG chains.

In a recent study the immediate and long-term effects of a single injection of chondroitinase ABC on CS-PG, GAG and axon regeneration were analyzed (Lin R et al. J. Neurochem. 104, 400-408, 2008). In this study a single dose of chondroitinase ABC injected adjacent to the damaged adult nigrostriatal tract of the rat showed that this promoted axon regeneration to a similar extent as in the aforementioned previous studies.
In this study it was also shown that the active enzyme persisted in the injured CNS for at least 10 days and prevented the rise in GAG concentration that occurs normally following CNS damage.

Taken together, there is strong evidence for the important role of CS (-PG) for the impaired axonal regeneration after CNS injury (but also spinal cord and peripheral nerve injury).
The data described sofar, strongly suggest that enzymatic removal of the CS GAG side chains by chondroitinase ABC (or chondroitinase AC) will lead to improved regeneration of injured nerve. fibres, increased plasticity and recovery of nerve function.

Therefore, the use of enzymes that degrade CS at the site of CNS and SC injury might represent an interesting novel therapeutic approach.

Application EP 1 911 460 A1 describes the use of chondroitinase ABC for promoting axonal regrowth and behaviour recovery in spinal cord injury.

Application US20007274979 describes the use of chondroitinase AC and/or chondroitinase B for the degradation of CS to promote neuronal outgrowth.
These patent applications [0022] and [0023] have to be seen in the light of the preceeding publications (see above).

However, a major problem for the use of chondroitinase ABC (or chondroitinase AC) in the human will come from the fact that chondroitinase ABC is a bacterial enzyme (purified from extracts of *Proteus vulgaris;* Yamagata T et al., J. Biol. Chem. 243, 15231535, 1968).

Therefore, injection of chondroitinase ABC to the site of injury will lead to an immune reaction against the bacterial enzyme, for example to the generation of anti-chondroitinase-ABC antibodies and as a consequence to a loss of enzyme activity. This will become even worse after repeated injections.

Furthermore, it cannot be excluded, that the immune reaction (and/or inflammation) leads to a severe damage of nerve tissue near the site of injection in the injured areas. Therefore, nerve tissue, that has not been affected by the primary injury, may be damaged, too.

Therefore, the availability of a **human endoglycosidase** that acts like the bacterial enzyme chondroitinase ABC and degrades chondroitin sulfate would be extremely valuable.

### Methods for the determination of the hyaluronidase and/or chondroitinase activity of hyaluronidases

The present invention relates also to methods for the determination of the hyaluronidase and/or chondroitinase activity of hyaluronidases. These methods are suitable not only for the measurement of the enzyme activity but also for screening of hyaluronidase/chondroitinase inhibitors (see below). Therefore, further applications of the methods, for example in the field of joint diseases but also of tumor therapy, will be described briefly.

CS GAG chains (see [0007]) are important components of the glial scar (see above [0006], [0008]-[0009]), however they are also widely distributed in the ECM of various tissues. For example, CS are the main GAG chains of aggrecan, the large aggregating proteoglycan of human cartilage.

Aggrecan is a major component of articular cartilage, where it is embedded in a network of collagen type II fibrils and is bound to hyaluronan chains via the HA-binding domain. The aggrecan molecule is built up by a large core-protein to which about 100 CS and up to 60 keratan sulfate (KS) GAG chains are attached.

In the course of inflammatory or degenerative joint diseases, aggrecan is degraded by a concerted action of different enzymes, for example metalloproteases and aggrecanase, released by inflammatory cells but also by chondrocytes.
However, in this process degradation of the CS chains of aggrecan and hyaluronan by the action of hyaluronidases might be an important mechanism, too, that contributes to the progressive destruction of articular cartilage in joint diseases.

Therefore, human hyaluronidases, that can degrade hyaluronan but also CS, might become interesting targets for the development of novel therapeutic approaches, for example hyaluronidase/chondroitinase inhibitors that can be used for the treatment of inflammatory joint diseases and osteoarthrosis.

The association of hyaluronan (see below [0051]-[0054]) with tumorigenesis has been known for some time. For example, hyaluronan and hyaluronidase are over-produced in many human tumors. Hyaluronan promotes cell invasiveness and some of the breakdown products of hyaluronan, released by the action of hyaluronidase, stimulate angiogenesis. Therefore, for example inhibitors of hyaluronidase activity might become a novel approach in the treatment of cancer (for review see Toole BP. Nature Rev. Cancer 4, 528-539, 2004).

Whereas the catabolic activity of bacterial chondroitinase (for example the chondroitinases ABC, AC-I, AC-II, B and C) can be measured easily by tracking the generation of the unsaturated bond by the lyase reaction (see [0013]), no rapid and sensitive assay has been described for measuring activity of vertebrate and especially human chondroitinase enzymes that use a hydrolase mechanism. In fact, only one assay has been described that is based on the competition of chondroitin sulfate with immobilized hyaluronan for the binding of TSG-6 protein (see below [0126]-[0127]), Wisniewski HG et al, Anal. Biochem. 347, 42-48, 2005; Wisniewski HG et al, J. Biol. Chem. 280, 14476-14484, 2005). However, this assay is not very sensitive (see below [0133]-[0134]).

In contrast, different methods for the determination of hyaluronidase activity have been described in the past. They can be divided into biological, physicochemical and chemical methods. However, these methods have in general a low accuracy and sensitivity and are not suitable for the use in high throughput screening (HTS).

The spreading assay, that measures the effect of hyaluronidase on the spreading of an indicator dye injected into the skin of animals, is an example of the biological assays.

Physicochemical methods have been based for example on the reduction in viscosity of a solution of hyaluronan (hyaluronic acid) after treatment with hyaluronidase. Such an assay is used for the standardisation of hyaluronidase preparations (quantified in terms of International Units (IU)) according to the European Pharmacopoeia (2002). However, the sensitivity of this assay is low and this assay is not suitable for HTS screening.

Alternatively, turbimetric assays are used for the measurement of hyaluronidase acitivity. In these assays, for example high molecular weight hyaluronan precipitates with diluted acidified serum, whereas depolymerized hyaluronan solutions remain clear.
Other turbidimetric assays are based on the formation of insoluble complexes of high molecular weight hyaluronan and for example cetylpyridinium chloride.

In turbidimetric assays hyaluronidase activity is expressed in turbidity reduction units (TRU) and 1 TRU is defined as the amount of enzyme which will reduce the turbidity produced by 0,2 mg of hyaluronan to that of 0,1 mg hyaluronan within 30 min.
A turbidimetric method is used for standardization of hyaluronidase according to the United States Pharmacopoeia (USP, 2002). The International Unit (IU) of hyaluronidase was defined as the activity of 0,1 mg of an international standard preparation (which is equal to 1 TRU).
However, again sensitivity and specificity of these assays are low and they are not suitable for HTS screening.

Chemical methods for the measurement of hyaluronidase acitivity are based on the determination of reducing sugars, e.g. N-acetyl-glucosamine resulting from the cleavage of the glycosidic bond of hyaluronan, by a colorimetric method (for example the Morgan-Elson-Assay). In these assays, hyaluronidase acitivity is quantified according to the International Union of Biochemistry, e.g. 1 Unit is the amount of enzyme that catalyses the release of 1 µmol of N-acetyl-glucosamine from the reducing end of sugars per min. However, again these assays have a low sensitivity and specificity and are not suitable for HTS screening.
Furthermore, chromatography, capillary zone electrophoresis, polyacrylamide gel electrophoresis and zymography have been used for the determination of hyaluronidase activity.

Recently, assays using flurogenic hyaluronan as substrate have been reported (Nagata H et al, Anal. Biochem. 330. 356-358, 2004) and ELISA-like assays, that are based on the use of a hyaluronan binding peptide (derived from aggrecan) (Frost Gl, Anal. Biochem. 251, 263-269, 1997, Wisniewski HG et al, Anal. Biochem. 347, 42-48, 2005).

### DETAILED DESCRIPTION OF THE INVENTION

The present invention relates to the use of members of the human hyaluronidase family (endo-beta-acetyl-hexosaminidase enzymes, E.C. 3.2.1.35) for the degradation of chondroitin sulfate (proteoglycans) to promote axonal regrowth in CNS or SC (spinal cord) injury.

As outlined in the previous section (FIELD AND STATE OF THE ART), there is strong evidence for the important role of CS (-PG) in the impaired axonal regeneration after CNS injury (but also spinal cord and peripheral nerve injury). Furthermore, it has been shown by a large number of in-vitro and in-vivo experiments that degradation of CS in the injured area by the bacterial enzyme chondroitinase ABC (and/or chondroitinase AC) will lead to improved regeneration of injured nerve fibres, increased plasticity and recovery of nerve function.

However, the use of the **bacterial enzyme chondroitinase ABC** (and/or chondroitinase AC) has major risks for human patients and therefore cannot be applied in the human safely (see above, [0024]-[0026]).

Therefore, the availability of a **human enzyme,** that acts like the bacterial enzyme chondroitinase ABC, e.g. can degrade chondroitin sulfate, would be extremely valuable.

The present invention relates to the use of human endo-beta-acetyl-hexosaminidase enzymes and in particular of the hyaluronidase type for degradation of chondroitin sulfate.

Hyaluronidases are enzymes capable of cleaving hyaluronan glycosaminoglycans (HA). According to their catalytic mechanism hyaluronidases were classified into three major families (Meyer, 1971, Hyaluronidases. in: The Enzymes, Vol V. ed Boyer PD, New York).

Human hyaluronidases belong to the first group (vertebrate hyaluronidases), whereas the other two groups comprise bacterial and parasitic hyaluronidases.

Human hyaluronidases (E.C. 3.2.1.35) are a group of five endo-beta-acetyl-D-hexosaminidase enzymes (Hyal-1, -2, -3, -4 and PH-20; and one pseudogene HYAL-P1).

Hyaluronidases degrade hyaluronan by a hydrolytic mechanism. They cleave the β-(1->4) glycosidic bond of hyaluronan by an endolytic process (see below [0069]).

Hyaluronan (previously called also hyaluronic acid) is a linear, negatively charged, high molecular weight glycosaminoglycan (GAG), that is found predominantly in the extracellular matrix (ECM) (Meyer K, Palmer JW, J.Biol.Chem. 107, 629-634, 1934; Fraser JRE, Laurent TC, in Extracellular Matrix, ed Comper WD, Amsterdam 1996).

Hyaluronan is composed of repeating disaccharides of β-D-glucuronyl-(1->3)-N-acetyl-D-glucosamine. These disaccharides are then linked by β-(1->4) glycosidic bonds.
Hyaluronan chains in the ECM can reach sizes of 100 - 10.000 kDa, however, they are not attached to a core-protein. Furthermore, hyaluronan chains are, in contrast to other GAG chains, not sulfated.

The structure and function of hyaluronan (Fraser JRE, Laurent TC, in Extracellular Matrix ed Comper WD, Amsterdam 1996) but also the catabolism of hyaluronan have been extensively studied (Stern R, Glycobiol. 13, 105-115, 2003).

Hyaluronan plays an important role in many different biological functions, including cell migration, invasion, differentiation and proliferation, for example by the interaction with hyaluronan-binding proteins and receptors (Fraser JRE, Laurent TC, in Extracellular Matrix ed Comper WD, Amsterdam 1996; Knudson CB, Knudson W. FASEB J. 7, 12331244, 1993, Toole BP et al., J. Biol. Chem. 277, 45934596, 2002).

In addition to the degradation of hyaluronan, vertebrate hyaluronidases are capable to catabolize chondroitin, chondroitin-4-sulfate, chondrotin-6-sulfate and dermatan sulfate as alternative substrate (Meyer K, Rapport MM, Adv. Enzymol. Relat. Areas Mol. Biol. 13, 199-236, 1952), although at a slower rate (see below, example 5 [0162]-[0167]).

The cleavage of chondroitin sulfate by vertebrate hyaluronidases is not a non-specific reaction but can be explained by the close relationship of chondroitin sulfate and hyaluronan. Comparison of the basic disaccharide subunit of both glycosaminoglycans, e.g. chondroitin sulfate and hyaluronan, shows that both glycosaminoglycans contain glucuronic acid as uronic acid. Furthermore, the N-acetyl-glucosamine residue in hyaluronan is the C-4 epimer of the N-acetyl-galacatosamine residue in chondroitin sulfate (e.g. they differ only in the configuration at C-4 with respect to the orientation of the hydroxyl group). Even though in chondroitin sulfate these disaccharides can be substituted to a varying degree with sulfate groups, it is evident that hyaluronan is quite similar to chondroitin sulfate.

Previously, two forms of hyaluronidase with different pH optimums have been described, one with an acidic and one with a neutral pH optimum. The sperm protein PH-20 was identified to be a hyaluronidase with neutral pH optimum.
Based on their homology to PH-20 further hyaluronidases, like Hyal-2, have been identified.

Human plasma hyaluronidase has been purified and its cDNA was cloned in 1997 (Frost et al.Biochem. Biophys. Res. Commun. 236, 10-15). The gene encoding this enzyme, now called HYAL1, was mapped to Chromosome 3p21.3.

PH-20, Hyal-1 and Hyal-2 differ with respect to the degradation of hyaluronan. Whereas hyaluronan is cleaved by hyaluronidase-2 to 20 kDa intermediate-size fragments (of about 50 disaccharides), by PH-20 and hyaluronidase-1 hyaluronan is cleaved down to disaccharides or tetrasaccharides (Stern R, Glycobiol. 13, 105-115, 2003, Hofinger et al. Glycobiology 17, 963-971, 2007, Hofinger et al. Glycoconj. J. 25, 101-109, 2008).

In the human, three genes (HYAL1, HYAL2, HYAL3) are found tightly clustered on chromosome 3p21.3 coding for hyaluronidase-1 (hyal-1), hyal-2 and hyal-3. Another three genes HYAL4, PHYAL1 (a pseudogene) and SPAM1 (Sperm Adhesion Molecule 1) are clustered in a similar fashion on chromosome 7q31.3. They code for Hyal-4 and PH-20 (Csoka AB, et al. Matrix Biol. 20, 499-508, 2001; Csoka AB et al., Genomics 60, 356-361, 1999).

Cloning of the mammalian hyaluronidases has facilitated a detailed analysis of their structure and function and their unique mechanism of hydrolysis (Jedrzejas MJ, Stern R. Proteins 61, 227-238, 2005; Stern R. Glycobiol. 13, 105-115, 2003; Chao KL, et al. Biochem. 46, 6911-6920, 2007).

Sequence analysis of the cloned enzymes (Hyal-1-4 and hPH-20) has shown that theses hyaluronidases are relative uniform in their size (chain length ranging from 435 amino acids (aa) (hyal-1) to 510 aa (hPH-20)).

Pairwise and multiple sequence alignments of these hyaluronidases have shown sequence identities ranging from 33 % to 41 % and indicate common structural properties. Furthermore, there are a number of absolutely conserved regions (Jedrzejas MJ, Stern R. Proteins 61, 227-238, 2005), that are necessary for the function of hyaluronidases (see below, [0065]-[0067]).

Although X-ray crystal structures are available at present only for bee venom hyaluronidase (BvHyal) and bovine PH-20 (bPH-20), based on their homology to the human hyaluronidases, these enzymes are thought to be structurally representative for all vertebrate hyaluronidases (Jedrzejas MJ, Stern R. Proteins 61, 227-238, 2005).

For all human hyaluronidases (Hyal-1-4 and hPH-20) a two domain structure was shown. The major domain, that has catalytic activity, starts from the N-terminus of the protein and is followed by a smaller C-terminal domain.

The active site was identified based on the homology to BVHyal and mutational analysis (Jedrzejas MJ, Stern R. Proteins 61, 227-238, 2005). The active site is located within the substrate-binding cleft of the catalytic domain. This cleft is surrounded by a number of highly conserved positively charged and hydrophobic amino acid residues that correspond to the negatively charged and hydrophobic substrate, e.g. hyaluronan or chondroitin sulfate.

Within the catalytic site, a single catalytic residue, Glu131 (numbering according to Hyal-1), has been identified, that acts as a proton donor, and a group of amino acid residues that position the substrate, e.g. Asp129, Tyr202, Tyr247, and Trp321, (numbering according to Hyal-1) (Jedrzejas MJ, Stern R. Proteins 61, 227-238, 2005). These residues are strictly conserved for all human hyaluronidases (Hyal-1-3 and hPH-20) with exception of Hyal-4, where the Tyr247 amino acid residue (Hyal-1 numbering) is replaced by Cys263 (Hyal-4 numbering), strongly suggesting, that this replacement is responsible for the different specificity of Hyal-4, compared to the other hyaluronidases, e.g. Hyal-4 has no hyaluronidase activity but a chondroitinase activity (see below, [0077]-[0079], [0081]).

A possible explanation is, that the gene for Hyal-4 is closely related to the ancestral sequence, that was specific for chondroitin and/or chondroitin sulfate. In fact, in *Caenorhabditis elegans,* there is only one sequence with homology to hyaluronidase and the only GAG detected was chondroitin, which is mostly unsulfated.

The catalytic mechanism of the hyaluronidases involves double displacement at C1 next to the β-(1->4) glycosidic bond to be cleaved. Concomitant with cleavage of the glycosidic bond, a glutamic acid (Glu131 in hHyal-1) transfers a proton to the C-4 oxygen of the released hyaluronan fragment. A water molecule replaces the released hyaluronan fragment and completes hydrolysis.

Furthermore, under specific conditions (especially at high substrate concentrations) hyaluronidases may also have a transglycosidase activity.

Whereas Hyal-1 has been described first as a plasma hyaluronidase, three members of the hyaluronidase family are GPI-anchored to the cell membrane, e.g. Hyal-2, Hyal-4 and PH-20. However, at least for PH-20 a proteolytically processed soluble form has been described and probably soluble forms are generated by the same mechanism for the other hyaluronidases.

In summary, **vertebrate and** especially **human hyaluronidases use a highly conserved mechanism for the degradation of hyaluronan and also of chondroitin** sulfate and therefore play an important role also in the catabolism of chondroitin sulfate (proteoglycans). This is clearly shown for vertebrate hyaluronidase by the experiment given in example 5 (see below [0162]-[0167].

Therefore, these **human enzymes,** that act like the bacterial enzyme chondroitinase ABC e.g. they can degrade chondroitin sulfate (see examples 4 and 5 [0156]-[0167]), could become extremely valuable for the treatment of CNS and spinal cord injury (see above), although the mechanism of cleavage is different for hyaluronidases (hydrolases) and chondroitinase ABC (lyases) (see above [0069] and [0013]).

Whereas the use of the **bacterial enzyme chondroitinase ABC** has major risks for human patients and therefore cannot be used in the human (see above, [0024]-[0026]), these restrictions (for the use in the human) do not apply for human hyaluronidases (see above [0027]).

The present invention relates to the use of the members of the human hyaluronidase family for the degradation of chondroitin sulfate (proteoglycans) in the glial scar to promote axonal regrowth in CNS or spinal cord injury.

The present invention relates in particular to the use of those members of the human hyaluronidase family that have chondroitinase activity. Sofar these members are Hyal-1, Hyal-2, Hyal-4 and PH-20, whereas for Hyal-3 the enzymatic activity has to be clarified.

Whereas, Hyal-1 Hyal-2 and PH-20 have hyaluronidase activity in addition to chondroitinase activity, Hyal-4 has no hyaluronidase activity. Therefore, Hyal-1 Hyal-2 and PH-20 will degrade not only chondroitin sulfate (proteoglycans) but also hyaluronan, when injected into the glial scar or the site of CNS or spinal cord injury.

In contrast, Hyal4 has no hyaluronidase activity and will degrade specifically chondroitin sulfate, when injected into the glial scar or the site of CNS or spinal cord injury.

Therefore, according to the present invention both groups of enzymes can be used as a therapeutic agent in a pharmaceutical composition for the treatment of human CNS and spinal cord injury.
However, in a preferred embodiment of the invention members of the hyaluronidase family that have only chondroitinase but no hyaluronidase acitivity were used ([see [0077], [0078] and [0081]).

As the human hyaluronidases have been cloned, preferably recombinant enzymes are used, although in principal purified natural human hyaluronidases can be used as well.

Furthermore, as the difference in the substrate specifity of Hyal-4, compared to the other hyaluronidases, could be most probably attributed to a replacement of the Tyr247 amino acid residue (Hyal-1 numbering) by Cys263 (Hyal-4 numbering) (see above [0067]), it will be easily possible to modify the specificity of the other hyaluronidases for example by site directed mutagenesis and to generate further enzymes, derived from human hyaluronidases, that have preferential or exclusive chondroitinase activity.

In addition, enzymes of the human hylauronidase family could be further modified by various techniques to improve their properties. These techniques comprise on the one hand a variety of molecular biology techniques, like site directed mutagenesis, that are well known to those skilled in the art, on the other hand they comprise also methods of protein and carbohydrate chemistry. By these techniques, for example fragments of human hyaluronidase enzymes could be generated, that possess only the catalytic domain or contain no GPI-anchor. Furthermore, glycosylation of the enzymes could be modified, and thereby, for example the stability but also the activity of the enzymes.

Whether the hyaluronidase activity of Hyal-1, Hyal-2 and PH-20, in addition to their chondroitinase activity, might be an advantage for the treatment of CNS or spinal cord injury has to be tested. Studies of embryonic development, regeneration as well as cancer have shown, the extracellular matrices surrounding proliferating and migrating cells are highly enriched in hyaluronan (Toole BP, Semin. Cell Dev. Biol. 12, 79-87, 2001; Toole BP et al., J. Biol. Chem. 277, 4593-4596, 2002). This could indicate, that degradation of hyaluronan will inhibit cell proliferation and migration. However, this might be different in CNS and spiral cord for axon sprouting, and degradation of hyaluronan in the extracellular matrix might facilitate axon sprouting (Moon LD, et al., Neurosci. Res., 71, 23-37, 2003).

Furthermore, if necessary the ratio of the chondroitinase activity/hyaluronidase activity could be adjusted easily. This could be achieved either by modification of the enzyme (see above [0082]) or more easily in a pharmaceutical composition by the use of a mixture of at least two of the (recombinant) enzymes, that contain either additional hyaluronidase activity (for example Hyal-1) or not (for example Hyal-4).

The pharmaceutical composition according to the invention may be administered by different ways that are suitable to reach the desired site, e.g. the injured tissue. Preferably the administration will be by injection into the site of injury or by infusion, for example via an epidural intrathecal catheter.

For the purpose of administration, the hyaluronidase enzymes according to the invention are used in a formulation suitable for the respective kind of administration using corresponding pharmaceutical excipients.

### Methods for determining endoglycosidase enzyme activity, and in particular of the hyaluronidase/chondroitinase type

The present invention also relates to methods for determining endoglycosidase enzyme activity, and in particular of the hyaluronidase/chondroitinase type (see above), in a sample and in one preferred embodiment to a method for detecting compounds capable of modulating the activity of an endoglycosidase, in particular of an endoglycosidase having activity either of the hyaluronidase or the chondroitinase type (see above).

The invention relates in particular to an assay for hyaluroindase/chondroitinase (format a), where the substrate is labelled with one member of a ligand-receptor pair. The corresponding member of the ligand-receptor pair will generate, either directly or indirectly (see [0097]-[0098]), the signal to be measured. In one preferred embodiment of the method, compounds to be tested for their chondroitinase modulating activity were incubated in the first step together with a member of the hyaluronidase enzyme family (that has chondroitinase acitivity) and the labelled substrate (see below [0106]).

However, as the active site of hyaluronidase enzymes is the same for both substrates, e.g. hyaluronan and chondroitin sulfate (see above [0063]-[0069]), this assay can also be used to measure hyaluronidase activity against hyaluronan or hyaluronan-derived substrates. This assay can also be used to screen for inhibitors of the hyaluronan-degrading acitivity of hyaluronidases, e.g. of hyaluronidase inhibitors.
Therefore, in another preferred embodiment of the method, compounds to be tested for their hyaluronidase modulating activity were incubated in the first step together with a member of the hyaluronidase enzyme family (that has hyaluronidase acitivity) and the substrate (hyaluronan or hyaluronan-derived molecules). In the second step, the substrate hyaluronan will compete with labelled chondroitin sulfate for binding to immobilized binding protein.
Therefore, in the following, to avoid unnecessary repetitions and redundancies, the assay is described primarily for the chondroitinase activity.

In this assay the labelled substrate in solution (see [0129]) is cleaved by hyaluronidase in a dose-dependent manner. Depending on the size of the resulting fragment, the cleaved labelled substrate does no longer bind to a binding protein immobilized on a solid phase support, that in a preferred embodiment of the method is the polycationic protein protamine. The use of protamine as CS binding protein (CS-BP; or hyaluronan-binding protein HA-BP) has several advantages compared to other CS-BP or HA-BP, like TSG-6 etc. (see [0126]-[0128]).

In this assay protamine can in particular be a protamine purified from sperm of different species, like salmon, herring, etc., a recombinant protamine, a low molecular weight protamine obtained for example after proteolytic cleavage of protamine, or a protamine-peptide. Furthermore, different protamine-like proteins, a class of arginine-rich proteins that belong to the histone family, can be used in this assay (Lewis, J.D. et al., Chromosoma 111: 473-482, 2003).

In the presence of a compound which inhibits (or activates) endoglycosidase activity, in particular activity of the chondroitinase/hyaluronidase type, the measured signal will be modified compared to the signal measured in the absence of this compound.

In order to develop an assay for endoglycosidase enzyme activity, and in particular enzyme activity of the hyaluronidase/chondroitinase type, the inventor had to overcome technical difficulties specifically encountered when assaying endoglycosidases, and in particular of the hyaluronidase/chondroitinase type.

The substrates of endoglycosidases, and in particular of the hyaluronidase/chondroitinase type, and especially CS are heterogeneous both with regard to the sequence of the disaccharide units (and their respective modifications) and the length of the chains.

The invention relates to a method for determining endoglycosidase enzyme activity comprising the following steps:
i. bringing a labelled substrate of an endoglycosidase in solution into contact with said endoglycosidase under conditions sufficient for degradation of the substrate by the endoglycosidase
ii. separating degradation products from undegraded or partially degraded substrate by binding the undegraded or partially degraded substrate (see [0090]) to a solid phase bound CS-BP (or HA-BP), preferably to the polycationic protein protamine
iii. measuring the change in the amount of intact labelled substrate, a decrease in the amount of this substrate being representative of endoglycosidase activity in the sample.

After degradation of the labelled substrate by the endoglycosidase, and in particular by an enzyme of the hyaluronidase/chondroitinase type, degraded substrate will no longer bind to the binding protein (see [0090]), that in a preferred embodiment of the method is the solid-phase bound polycationic protein protamine. After removal of unbound substrate by washing, bound labelled substrate can be measured. In this method, the substrate is labelled directly or indirectly.

The term "direct labeling" is intended to mean attachment of a label, for example a fluorescent label, to a functional group present on, or previously introduced onto, or generated on the substrate or on at least one of the members of the ligand/receptor pair. A spacer can be introduced between the label and the substrate or at least one of the members of the ligand/receptor pair.

The term "indirect labeling" is intended to mean attachment of the label, to the substrate via the second member of a ligand/receptor pair. The label is selected either from the group of enzymes, that are in use for enzyme immuno assays, like peroxidase, alkaline phosphatase etc, or from the group of fluorescent, chemiluminescent, electrochemiluminescent or colorimetric labels.

In one preferred embodiment of this method, the substrate is labelled indirectly with biotin via the peptide stub of the peptido-CS (see [0106]-[0107) and avidin coupled to peroxidase is used as the second member of the ligand receptor pair.

This method can be used to measure the activity of endoglycosidases capable of cleaving hyaluronan and chondroitin sulfate, such as, for example, hyaluronidase.

The method for determining endoglycosidase enzyme activity described above may make it possible to study the effects of modulation of this enzyme activity, exerted by compounds for which the testing of the influence on the enzyme activity is desired.

The expression "modulation of enzyme activity" is intended to mean inhibition or activation of this enzyme activity, regardless of the mechanism.

In one preferred embodiment the invention therefore relates to a method for detecting
a compound capable of modulating enzyme activity of the endoglycosidase type, comprising the following steps:
i. bringing a substrate of an endoglycosidase in solution into contact with an endoglycosidase, in the presence or absence of the test compound,
ii. measuring the change in the amount of intact substrate by time, and
iii. comparing the change in the amount of substrate by time measured in the absence of the test compound with that measured in the presence of the test compound.

In this method, the endoglycosidase substrate and/or at least one of the other components can be directly or indirectly labelled as described above (see [0097] - [0098]).

In the latter method, the endoglycosidase used can in particular be a hyaluronidase chosen from vertebrate hyaluronidases, preferably human hyaluronidases. This enzyme can be a recombinant hyaluronidase, a purified hyaluronidase or a non-purified hyaluronidase. The enzyme is usually provided in at least a partially purified form.

The substrate used in the preceeding methods can be selected from chondroitin sulfate proteoglycans (CS-PG) and peptido-chondroitin-sulfates (peptido-CS), synthetic oligosaccharides or oligosaccharides derived from CS or their derivatives. Peptido-CS are derived from CS-PG after proteolytic degradation of the core protein, for example by papain, resulting in a CS GAG chain with a short residual peptide-stub.

Substrate derivatives can be CS or CS-PG which have undergone minor modifications such as for example, but not limited to, selective desulfatation, graded N-ace-tylation, or reductive oxyamination, that do not interfere with the enzyme-substrate recognition, i.e. they can be cleaved by an enzyme having activity of the hyaluronidase/chondroitinase type (Naggi A. et al, J. Biol. Chem. 280 : 12103-12113, 2005, Fernandez C. et al., Carbohydrate Res. 341: 1253-1265, 2006; Sandbäck-Pikas D, et al., J. Biol. Chem 273: 18770-18777, 1998, Ramsay S., et al. Carbohydrate Res. 333: 59-71, 2001).

Alternatively hyaluronan and/or hyaluronan-derived fragments or synthetic oligosaccharides can be used as substrate (see above [0069]).

The method according to the invention can be implemented using various formats. The following formats are the preferred formats.

Format [a]: the substrate is covalently attached to a first member of a ligand/receptor pair. The other member of the ligand receptor pair is coupled to an enzyme detection system, like the peroxidase system.

Format [b]: the substrate is covalently attached to a first member of a ligand/receptor pair. The other member of the ligand/receptor pair is labelled for example with a fluorescent label.

Format [c]: the substrate is covalently attached to a fluorescent, chemoluminescent or electrochemiluminescent label. In this format the signal from the labelled substrate bound to the solid phase is measured directly.

The "ligand-receptor pair" denotes two binding partners such as the pairs: biotin/avidin; biotin/streptavidin; hapten/antibody systems like DNP (dinitrophenol)/anti-DNP antibody or HAG (influenza hemagglutinin peptide of 9 amino acids)/anti-HAG antibody; GST (glutathione S-transferase)/anti-GST antibody; 6HIS (peptide consisting of 6 histidines)/anti-6HIS antibody; c-myc (peptide of amino acids 410-419 of the human c-myc protein)/anti-c-myc antibody, or FLAG(R) (peptide of 4 amino acids)/anti-FLAG (R) antibody. Other ligand-receptor pairs known to those skilled in the art can be used.

These "ligand/receptor" systems are well known to those skilled in the art and are mostly commercially available.

The member of the ligand-receptor pairs can be covalently attached to the substrate using a variety of reactional groups such as hydroxysuccinimide ester or hydroxysulfosuccinimide esters.

The method for detecting a compound capable of modulating enzyme activity of the hyaluronidase/chondroitinase type makes it possible to screen large libraries of products which can in particular be anti-hyaluronidase antibodies, natural products, synthetic products, products from a library of compounds obtained by combinatorial chemistry, for example of peptides and proteins for their ability to modulate the enzyme activity.

Furthermore, the invention also relates to kits, containing the reagents usable or required to carry out the embodiments of the method according to the invention, and in particular the following elements:
- A substrate which can be cleaved by an enzyme having activity of the hyaluronidase/chondroitinase type, in particular CS-(GAG) or peptido-CS (GAG) preferably labelled with a first member of a receptor/ligand pair (see [0113])
- Hyaluronidase (i.e. vertebrate or human recombinant hyaluronidase, purified hyaluronidase or non-purified hyaluronidase)
- A solid-phase bound CS-BP (or HA-BP)
- A detection system comprising the second member of the receptor ligand pair

A kit according to the invention (to measure hyaluronidase/chondroitinase
activity) preferably contains:
- The substrate chondroitin sulfate (peptido-CS) labelled with biotin (via the peptid
   stub) (see [0106])
- Avidin (or streptavidin) coupled to peroxidase and a suitable substrate like ortho-phenylendiamine (OPD)
- A protamine-coated solid phase, for example a microplate

In another embodiment a kit according to the invention (to test potential modulators of hyaluronidase/chondroitinase activity) preferably contains:
- The substrate chondroitin sulfate (peptido-CS) labelled with biotin (via the peptid
   stub) (see [0106])
- Avidin (or streptavidin) coupled to peroxidase and a suitable substrate like OPD
- Hyaluronidase (i.e. recombinant hyaluronidase, purified hyaluronidase or non-purified hyaluronidase)
- A protamine-coated solid phase, for example a microplate

The method according to the invention has many advantages compared to the methods of the prior art, and in particular:

The method is a non-radioactive assay.

The assay is very simple to carry out since it comprises only few steps and will be completed in less than 3 h.

The method does not require expensive technical equipment.

The enzymatic reaction of the endoglycosidase, and in particular of the hyaluronidase/chondroitinase type, with the labelled substrate is performed in solution and not with a substrate coupled to a solid phase. Therefore, problems with steric hindrance and a lack of accessability of cleavage sites on the substrate for the enzyme are avoided (see [0034], [0127]).

No chemical treatment or modification of the GAG-chain is required. In this assay format only the peptid stub (of the original core protein) is labelled by a small ligand, for example biotin. Therefore, hyaluronidase/chondroitinase cleavage sites of the CS (GAG) are readily accessible, as outlined above.

The method according to the invention preferably uses as CS-BP (or HA-BP) the polycationic protein protamine instead of other ligands for CS, for example of TSG-6 (TNF-stimulated gene 6) (see [0034], [0127]) (Wisniewski HG et al., J. Biol. Chem. 280, 14476-14484, 2005).

TSG-6 has only one binding domain, e.g. the Link module as binding domain for hyaluronan, that is shared with other hyaladherins (however, the C-terminal CUB domain might also affect the binding of TSG-6 to GAGs). The Link module of TSG-6 binds to five distinct GAGs, e.g. chondroitin-4-sulfate, dermatan sulfate, heparan sulfate, heparin and hyaluronan (Blundell CD et.al., J. Biol. Chem. 282, 12976-12988, 2007). Binding of these ligands to the binding domain, a hyaluronan binding groove, of the Link module of TSG-6 is strictly pH-dependent.

In contrast, the cationic groups, e.g. the arginine residues, that mediate the ionic interaction of the polyanion ic CS (or hyaluronan) and protamine, are more evenly distributed on protamine.

In summary, the assay according to the invention is more sensitive and less vulnerable to changes of the composition of the substrate, to changes of the pH and is not dependent on the orientation and accessability of a specific binding domain (see above [0090], [0125]).

In addition, the use of TSG-6 or other specific binding proteins for the labelled substrate will lead to high costs of the assay.

In contrast to an immunoassay, that uses for example monoclonal antibodies to different epitopes of the hyaluronidase (chondroitinase) molecule, in the assay according to the invention the functional activity of hyaluronidase/chondroitinase in a sample is measured instead of the enzyme concentration. In contrast, an immunoassay will detect, dependent on the assay design and the respective antibodies, also inactive or degraded hyaluronidase. Therefore, an immunoassay does not allow to analyse modulators of endoglycosidase activity.

The volumes used are very small (total volume of 100 µl per well, in example 4 and 5). However, the assay can be miniaturized further (for example using 384 well plates) and this makes it possible to save on reagents, for example :

As little as 10 ng/ml of substrate is used in example 4. In contrast, in the assay of Wisnieski (Wisniewski HG et al, Anal. Biochem. 347, 42-48, 2005; Wisniewski HG et al, J. Biol. Chem. 280, 14476-14484, 2005; see also the application US2007134228) chondroitin-4-sulfate was used at a concentration of 0.5 mg/ml.

The same is true for the amount of enzyme used in the assay. In example 4 less than 0.1 µU (highest concentration of the standard curve) of the enzyme hyaluronidase are used, whereas in the assay of Wisnieski (Wisniewski HG et al., Anal. Biochem. 347, 42-48, 2005) hyaluronidase at a concentration of 0.001 - 1.0 unit/ml (according to the USP reference standard) were used.

The incubation times for all steps are very short, as is shown in example 4, i.e. the enzyme reaction requires 1 h of incubation (but can be reduced further).

The method according to the invention therefore makes it possible to dertermine the hyaluronidase and chondroitinase activity of hyaluronidases in a sample or to rapidly screen libraries of substances capable of modulating heparanase activity.

Thus, for the embodiments of the method according to the invention, there are important applications in the field of in vitro diagnosis but especially in the field of high throughput screening (HTS) in the pharmaceutical industry.

Format [a] (see [0110]) used in the examples 1-5 is therefore entirely suitable for a method to measure endoglycosidase enzyme activity, and in particular of the hyaluronidase/chondroitinase type, but also for testing a modulator of this enzyme activity.

### BRIEF DESCRIPTION OF THE FIGURES

**Figure 1** : The bar graph shows the dose-dependent binding of biotin-labelled chondroitin sulfate to the CS-BP-coated solid phase.

**Figure 2** : The bar graph shows the dose-dependent degradation of biotin-labelled chondroitin sulfate by chondroitinase ABC.

**Figure 3** : The bar graph shows the dose-dependent degradation of biotin-labelled chondroitin sulfate by hyaluronidase

### EXAMPLES

The following examples illustrate in a nonlimiting manner the chondroitinase activity of hyaluronidase and some of the preferred embodiments of the methods according to the invention:

### Experimental Section

The following abbreviations are used:
CS: chondroitin sulfate
NHS: N-hydroxysuccinimide
PBS: phosphate buffered saline
POD: horseradish-peroxidase
OPD: orthophenylenediamine

For the examples, the substrate is functionalized using the NH₂ functions of the peptido-CS (peptido-CS is produced by digestion of core proteins of purified chondroitin sulfate proteoglycans with a proteolytic enzyme like papain, resulting in glycosamino-glycan chain with short peptide stubs).

### EXAMPLE 1

### Preparation of a -CS-Biotin Substrate

Reagents Used:
- Solution of CS at 5 mg/ml: 5 mg CS +1 ml PBS, that has been obtained by digestion of aggrecan, purified from human articular cartilage, by papain.
   (However, CS can be obtained also commercially, for example Sigma H9902)
- Solution of Sulfo-NHS-LC-Biotin (EZ-Link® Sulfo-NHS-LC-Biotin) at 20 mM:
   11 mg Sulfo-NHS-LC-Biotin (Pierce) + 1 ml PBS (Gibco)

Biotin Labeling
1 ml of a solution of CS at 5 mg/ml are mixed with 0.1 ml of 20 mM Sulfo-NHS-LC-Biotin. The mixture is incubated for 18 h at 37°C. Excess biotin is removed using a 5 ml Zeba™ Desalt Spin Column (Pierce), equilibrated with PBS. By this, 1 ml of solution containing the biotinylated CS, hereinafter referred to as biotin-CS, was obtained.

### EXAMPLE 2

### Determination of the Final Molar Ratios

Assaying the Biotin:

The biotin concentration is measured using a photometric assay based on the competition of biotin-CS and HABA (4'-hydroxyazobenzene-2-carboxylic acid) for binding to avidin. Absorbance of the HABA/avidin complex is measured at 500 nm. Subsequently, biotin-CS is added and the decrease of absorbance is recorded. Known amounts of biotin-labelled POD are used as standard to calculate the molar ratio of biotin and CS.

### EXAMPLE 3

### Testing the Binding of Biotin-labelled CS to a CS-BP (protamine)-coated Solid Phase

Reagents Used:
- Biotin-labelled CS (see above, example 1)
- Protamine sulfate, from herring, grade III, Sigma P4505
- Avidin-POD (Sigma A 3151)
- PBS/0.01 % Tween-20
- OPD (Dako S 204530)

A solid phase, for example a microtiterplate, is coated with protamine by incubation of 100 µl/well of a 0.003 % solution of protamine sulfate at 37 °C for 24 hours. Thereafter, plates were washed 3 times with A dest. and stored in the dark.

The binding capacity of the solid phase, e.g. the wells of the microplate, was analyzed by incubation of increasing amounts of biotin-labelled CS (0.1 -100 ng/ml) for 60 min at room temperature.

Plates were washed 3 times with buffer (PBS/0.01 % Tween 20) and Avidin-POD (1 µg/ml in PBS/5 % BSA) was added.

After 15 min plates were washed 5 times with PBS and 100 µl OPD-solution (0,667 mg/ml) was added.

After 15 min the reaction was stopped by addition of 100 µl of 0,5 Mol H₂SO₄. OD was measured at 490 nm using a microplate reader.

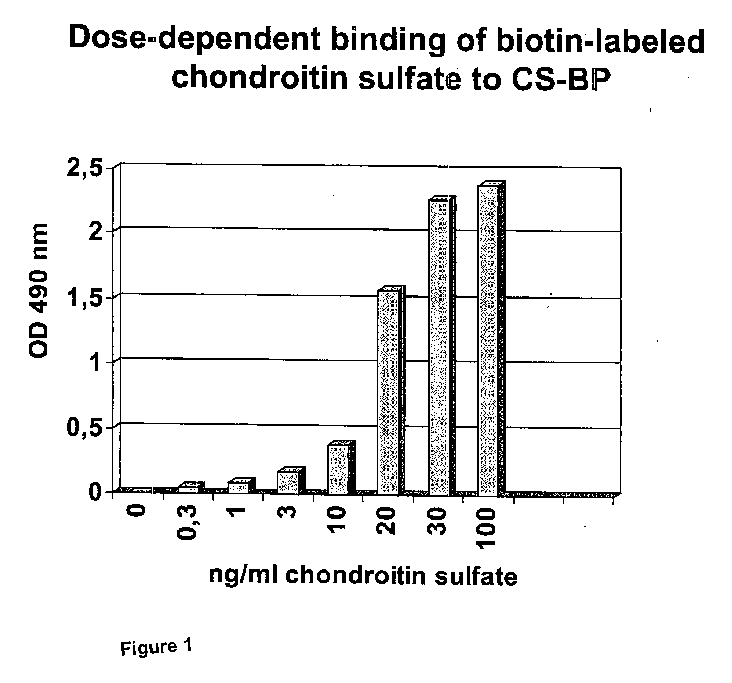

Fig. 1 shows the dose-dependent binding of biotin-labelled CS to the CS-BP (protamine)-coated solid phase.

### EXAMPLE 4

### Assaying Activity of the Chondroitinase Type (Chondroitinase ABC)

Reagents Used:
- same as in example 3
- Chondroitinase ABC from *Proteus vulgaris* (Sigma, C2905), according to the manufacturer one unit of chondroitinase ABC will liberate 1.0 µmole of 2-acetamido-2-deoxy-3-O-(β-D-gluc-4-ene-pyranosyluronic acid) from chondroitin sulfate C per min at pH 8.0 at 37°C.

The enzyme reaction is carried out by mixing 50 µl of biotin-labelled CS at 0.01 µg/ml with increasing amounts of chondroitinase ABC, e.g. 0,8-100 nano-units of chondroitinase ABC. Samples were brought to a volume of 100 µl by addition of PBS.

This mixture is incubated at 37°C for 1 h. Thereafter the enzyme was inactivated by heating at 100°C for 10 min.

Samples (85 µl of the samples) were transferred to the CS-BP (protamine)-coated microplate and incubated for 1 hour at room temperature. The following steps are identical to those described for example 3 ([0149]-[0155]).

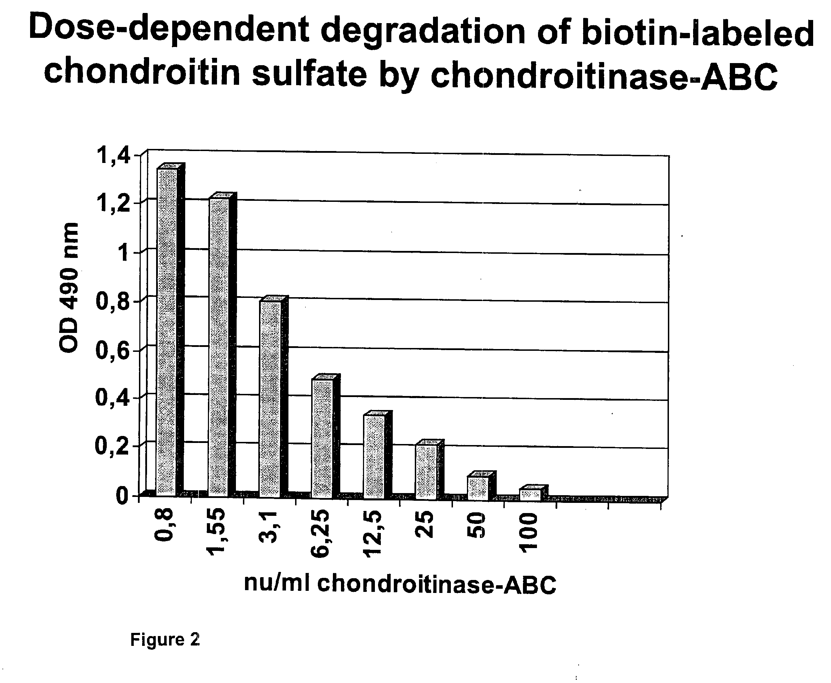

The results are expressed in FIG. 2, which shows the change in the signal for an increase in concentration of the enzyme chondroitinase ABC.

The decrease in the signal correlates perfectly with the increase in the enzyme activity, i.e. the cleavage of the biotin-labelled CS substrate.

### EXAMPLE 5

### Assaying Chondroitinase Activity of Hyaluronidase

Reagents Used:
- same as in example 3
- Testes hyaluronidase (type V, mol wt 55 kDa) from sheep (Sigma, C6254), according to the manufacturer one unit of hyaluronidase is based on the change of absorbance at 600 nm (change in turbidity) of a USP refence standard hyaluronidase.

The enzyme reaction is carried out by mixing 50 µl of biotin-labelled CS at 0.01 µg/ml with increasing amounts of hyaluronidase, e.g. 0,8 - 100 milliunits of hyaluronidase. Samples were brought to a volume of 100 µl by addition of PBS.

This mixture is incubated at 37°C for 1 h. Thereafter the enzyme was inactivated by heating at 100°C for 20 min.

Samples (85 µl of the samples) were transferred to the CS-BP (protamine)-coated microplate and incubated for 1 hour at room temperature. The following steps are identical to those described for example 3 ([0149]-[0155]).

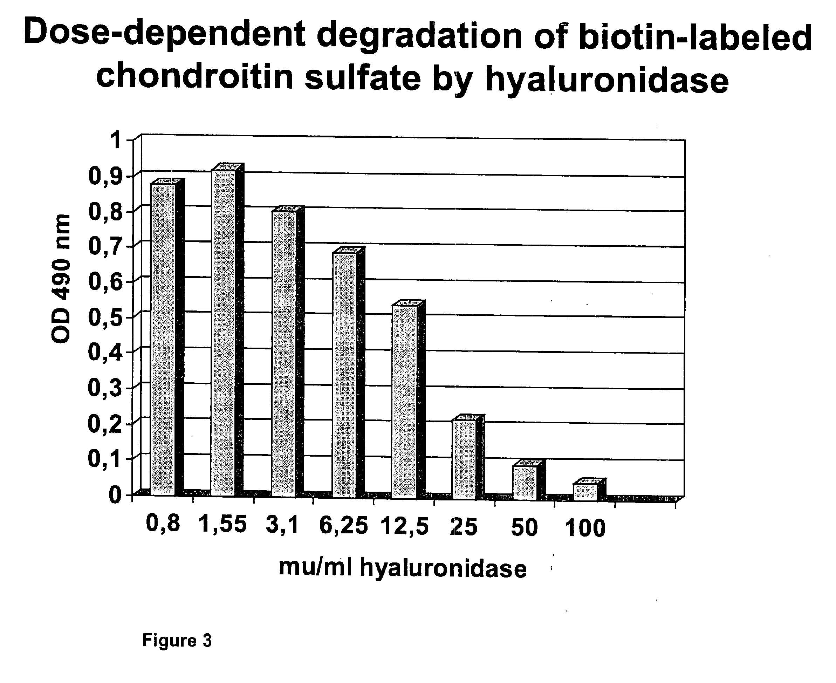

The results are expressed in FIG. 3, which shows the change in the signal for an increase in concentration of the enzyme hyaluronidase.

The decrease in the signal correlates perfectly with the increase in the enzyme activity, i.e. the cleavage of the biotin-labelled CS substrate.

### REFERENCES

Blundell CD, Mahoney DJ, Cordell MR, Almond A, Kahmann JD, Perczel A, Taylor JD, Camphell IS, Day AJ. 2007. Determining the molecular basis for the pH-dependent interaction between the link module of human TSG-6 and hyaluronan. J. Biol. Chem. 282, 12976-12988

Bradbury EJ, Moon LD, Popat RJ, King VR, Bennett GS, Patel PN Fawcett JW, McMahon SB. 2002. Chondroitinase ABC promotes functional recovery after spinal cord injury. Nature 416: 636-640

Busch SA, Silver J. 2007. The role of extracellular matrix in CNS regeneration. Curr. Opin. Neurobiol. 17, 120-127

Chao KL, Muthukumar L, Herzberg O. 2007. Structure of human hyaluronidase-1, a hyaluronan hydrolyzing enzyme involved in human tumor growth and angiogenesis. Biochemistry 46, 6911-6920

Chau CH, Shum DKY, Li H, Pei J, Lui YY, Wirthlin L, Chan YS, Xu XM. 2004. Chondroitinase ABC enhances axonal regrowth through Schwann cell-seeded guidance channels after spinal cord injury FASEB J. 18, 194-196

Crespo D, Asher RA, Lin R, Rhodes KE, Fawcett JW. 2007. How does chondroitinase promote functional recovery in the damaged CNS. Exp. Neurol. 206 : 159-171.

Csoka, AB, Frost Gl, Heng HHQ, Scherer SW, Mohapatra G, Stern R. 1998. The hyaluronidase gene HYAL1 maps to chromosome 3p21.2-p21.3 in human and 9F1-F2 in mouse, a conserved candidate tumor suppressor locus. Genomics, 48, 63-70

Csoka AB, Scherer SW, Stern R. 1999. Expression analysis of six paralogous human hyaluronidase genes clustered on chromosome 3p21 and 7q31. Genomics 60, 356-361

Csoka AB, Frost Gl, Stern R. 2001. The six human hyaluronidase-like genes in the human and mouse genomes. Matrix Biol. 20, 499-508

Curinga GM, Snow DM, Mashburn C, Kohler K, Thobaben R, Caggiano AO, Smith GM. 2007. Mammalian produced chondroitinase AC mitigates axon inhibition by chondroitin sulfate proteoglycans. J. Neurochem. 102, 275-288

Fawcett JW. 2006. The glial response to injury and its role in the inhibition of CNS repair. Adv. Exp. Med. Biol. 557: 11-24.

Fraser JRE, Laurent TC. 1996 Hyaluronan. pp 141-199 in Extracellular Matrix, ed Comper WD, Amsterdam

Frost Gl, Csoka AB, Wong T, Stern R. 1997. Purification, cloning, and expression of human plasma hyaluronidase. Biochem. Biophys. Res. Commun. 236, 10-15

Frost Gl, Stern R. 1997. A microtiter-based assay for hyaluronidase activity not requiring specialized reagents. Anal. Biochem. 251, 263-269

Glaser JH, Conradt HE. 1979. Chondroitin SO4 catbolism in chick embryo chondrocytes. J Biol. Chem. 254, 2316-2325

Groves ML, McKeon R, Werner E, Nagarsheth M, Maedor W, English AW. 2005. Axon regeneration in peripheral nerves is enhanced by proteoglycan degradation. Exp. Neurol. 195, 278-292

Hofinger ESA, Bernhardt G, Buschauer A. 2007. Kinetics of Hyal-1 and PH-20 hyaluronidases: Comparison of minimal substrates and analysis of the transglycosylation reaction. Glycobiol. 17, 963-971

Hofinger ES, Hoechstetter J, Oettl M, Bernhardt G, Buschauer A. 2008. Isoenzyme-specific differences in the degradation of hyaluronic acid by mammalian-type hyaluronidases. Glycoconj. J. 25, 101-109

Hoffman P, Linker A, Meyer K. 1958. Chondroitin sulfates. Fed. Proc. 17, 1078-1082

Houle JD, Tom VJ, Mayes D, Wagoner G, Phillips N, Silver J. 2006. Combining an autologous peripheral nervous system "bridge" and matrix modification by chondroitinase allows robust, functional regeneration beyond a hemisection lesion of the adult rat spinal cord. J. Neurosci. 26, 7405-7415.

Ingmar B, Wasteson A. 1979. Sequential degradation of a chondroitin sulfate trisaccharide by lysosomal enzymes from embryonic-chick epiphysial cartilage. Biochem. J. 179, 7-13

Jedrzejas MJ, Stern R. 2005 Structure of vertebrate hyaluronidases and their unique enzymatic mechanism of hydrolysis. Proteins. 61, 227-238

Jones LL, Sajed D, Tuszynski MH. 2003. Axonal regeneration through regions of chondroitin sulfate proteoglycan deposition after spinal cord injury: A balance of permissiveness and inhibition. J. Neurosci. 23, 92769288

Knudson CB, Knudson W. 1993. Hyaluronan-binding proteins in development, tissue homeostasis and disease. FASEB J. 7, 1233-1241

Lepperdinger G, Strobl B, Kreil G. 1998. HYAL2, a human gene expressed in many cells, encoding a lysosomal hyaluronidase with a novel type of specificity. J. Biol. Chem. 273, 22466-22470

Levine JM. 1994. Increased expression of the NG2 chondroitin sulfate proteoglycan after brain injury. J Neurosci. 14: 4716- 4730

Lin R, Kwok JC, Crespo D, Fawcett JW. 2008. Chondroitinase ABC has a long-lasting effect on chondroitin sulfate glycosaminoglycan content in the injured rat brain. J. Neurochem. 104, 400-408

Lu P, Jones LL, Tuszynski MH. 2007. Axonal regeneration through scars and into sites of chronic spinal cord injury. Exp. Neurol. 203, 8-21

Meyer K. Palmer JW 1934. The polysaccharide of the vitreous humor. J.Biol.Chem. 107, 629-634

Meyer K. 1971. Hyaluronidases. in: The Enzymes, Vol V. ed Boyer PD, Academic Press, New York

Meyer K, Rapport MM. 1952. Hyaluronidases. Adv. Enzymol. Relat. Areas Mol. Biol. 13, 199-236

Moon LD, Asher RA, Rhodes KE, Fawcett JW. 2001. Regeneration of CNS axons back to their target following treatment of adult rat brain with chondroitinase ABC. Nat. Neurosci. 4, 465-466.

Moon LD, Asher RA, Fawcett JW. 2003. Limited growth of severed CNS axons after treatment of adult rat brain with hyaluronidase. J. Neurosci. Res., 71, 23-37.

Nagata H, Kojima R, Sakurai K, Sakai S, Kodera Y, Nishimura H, Inada Y, Matsushima A. 2004. Molecular-weight-based hyaluronidase assay using fluorescent hyaluronic acid as a substrate. Anal. Biochem. 330, 356-358

Rhodes KE, Fawcett JW. 2004. Chondroitin sulfate proteoglycans: preventing plasticity or protecting the CNS ? J. Anat. 204: 33-48.

Sanderson PN, Huckerby TN, Nieduszynski IA. 1989. Chondroitinase ABC digestion of dermatan sulfate. Biochem. J. 257 : 347-354

Smith-Thomas L, Fok-Seang J, Stevens J, Du JS, Muir E, Faissner A, Geller HM, Rogers JH, Fawcett JW. 1994. An inhibitor of neurite outgrowth produced by astrocytes. J. Cell Sci. 107: 1687-1695.

Smith-Thomas L, Stevens J, Fok-Seang J, Muir E, Faissner A, Rogers JH, Fawcett JW. 1995. Increased axon regeneration in astrocytes grown in the presence of proteoglycan synthesis inhibitors. J. Cell Sci. 108: 1307-1315.

Snow DM, Lemmon V, Carrino DA, Caplan Al, Silver J. 1990. Sulfated proteoglycans in astroglial barriers inhibit neurite outgrowth in vitro. Exp. Neurol. 109 : 110-130

Shuttleworth TL, Wilson MD, Wicklow BA, Wilkins JA, Triggs-Raine B. 2002. Characterization of the murine hyaluronidase gene region reveals complex organitzation and cotranscription of Hyal1 with downstream genes Fus2 and Hyal3. J. Biol. Chem. 277, 23008-23018

Silver J, Miller JH. 2004. Regeneration beyond the glial scar. Nature Reviews Neuroscience 5, 146-156

Stern R. Jedrzejas MJ. 2006. Hyaluronidases: their genomics, structures, and mechanisms of action. Chem. Rev. 106, 818-839

Suzuki S, Saito H, Yamagata T, Anno K, Seno N, Kawai Y, Furuhashi T. 1968. Formation of three types of disulfated disaccharides from chondroitin sulfates by chondrotinase digestion. J. Biol. Chem. 243 : 1543-1550
Tan AM, Zhang W, Levine JM. 2005. NG2 : a component of the glial scar that inhibits axon growth. J. Anat. 207: 717-725

Toole BP. 2001. Hyaluronan in morphogenesis. Semin. Cell Dev. Biol. 12, 79-87

Toole BP, Wight TN, Tammi MI. 2002. Hyaluronan-Cell interactions in cancer and vascular disease. J. Biol. Chem. 277, 4593-4596

Toole BP. 2004. Hyaluronan : from extracellular glue to pericellular cue. Nature Rev. Cancer 4, 528-539

Ughrin YM, Chen ZJ, Levine JM. 2003. Multiple regions of the NG2 proteoglycan inhibit neurite growth and induce growth cone collapse. LiebJ. Neurosci. 23: 175-186

Wisniewski HG, Snitkin ES, Mindrescu C, Sweet MH, Vilcek J. 2005. TSG-6 protein binding to glycosaminoglycans. J. Biol. Chem. 280, 14476-14484

Wisniewski HG, Sweet MH, Stern R. 2005. An assay for bacterial and eukaryotic chondroitinases using a chondroitin sulfate-binding protein. Anal. Biochem. 347, 42-48

Yamagata T,Saito H, Habuchi O, Suzuki S. 1968. Purification and properties of bacterial chondroitinases and chondrosulfatases. J. Biol. Chem. 243 : 1523-1535

Zuo J, Neubauer D, Dyess K, Ferguson T A, Muir D. 1998. Degradation of chondroitin sulfate proteoglycan enhances the neurite-promoting potential of spinal cord tissue. Exp. Neurol. 154: 654-662

## Claims

1. Use of a vertebrate hyaluronidase polypeptide for the manufacture of a medicament for the treatment of a nervous system injury.

2. The use of claim 1 for the degradation of chondroitin sulfate (proteoglycans) to promote axonal regrowth in human CNS, spinal cord or peripheral nerve injury.

3. The use of claim 1 or 2, wherein the hyaluronidase is selected from mammalian, particularly from human hyaluronidases such as Hyal-1-4 and PH-20.

4. The use of any of claims 1-3, wherein the hyaluronidase is selected from natural proteins, recombinant proteins or low molecular weight forms or peptides of these proteins, derived for example by proteolytic cleavage.

5. The use of any of claims 1-4, wherein the hyaluronidase has been modified by molecular biology, protein chemistry or carbohydrate chemistry techniques like site directed mutagenesis methods.

6. The use of claim 5, wherein the hyaluronidase has been modified with respect to their hyaluronidase and/or chondroitinase acitivity, e.g. Hyal-1-3 and PH-20, where the Tyr247 amino acid residue (Hyal-1 numbering) is replaced by Cys263 (Hyal-4 numbering).

7. The use of any of claims 1-5, wherein hyaluronidase is a humanized form of a hyaluronidase protein from a non-human vertebrate, e.g. mammalian species.

8. A method for treating a subject with an injury of the nervous system, e.g. the CNS, spinal cord or peripheral nerve, comprising administration of an effective amount of at least one vertebrate hyaluronidase polypeptide as claimed in claim 1-7, to the site of the injured tissue.

9. The method of claim 8, wherein the hyaluronidase polypeptide is a mixture of hyaluronidases with different properties, for example enzymes that contain either additional hyaluronidase activity (for example Hyal-1, Hyal-2 and PH-20) or not (for example Hyal-4).

10. The method of claim 8 or 9 where the administration is by injection into the site of injury, by infusion, for example via an epidural intrathecal catheter, or by direct application to the site of injury.

11. The method of any one of claims 8-10 where the effective amount of hyaluroindase is administered by one or multiple doses at one or several days.

12. The method of any one of claims 8-11 1 where the hyaluronidase is administered in a formulation comprising suitable pharmaceutical excipients.

13. A method for determining endoglycosidase enzyme activity comprising the following steps:
(i) bringing an endoglycosidase substrate into contact with an endoglycosidase containing sample under conditions sufficient for degradation of the substrate by the endoglycosidase,
(ii) separating degradation products from undegraded or partially degraded substrate by binding the undegraded or partially degraded substrate to a solid phase bound chondroitin sulfate binding protein (e.g. CS-BP, or HA-BP), preferably to the polycationic protein protamine and
(iii) measuring the decrease of intact substrate relating to endoglycosidase activity in the sample,
wherein the substrate is directly labelled or indirectly labelled with a first member of a ligand/receptor pair, and the amount of intact substrate is determined by measuring a signal emitted by a detection system, bound to the substrate or to the second member of the ligand/receptor pair.

14. The method as claimed in claim 13, **characterized in that** the first member of the ligand/receptor pair is biotin and the second member of the ligand/receptor pair is avidin or streptavidin, e.g. coupled to an enzyme as part of the detection system, or that the ligand/receptor pair is selected from the hapten/antibody pairs such as DNP/anti-DNP antibody, GST/anti-GST antibody, 6HIS/anti-6HIS antibody; c-myc/anti-c-myc antibody; FLAG(R)/anti-FLAG(R) antibody; HAG/anti-HAG antibody.

15. The method as claimed in claim 13 or 14 wherein said sample is an extract of tissues or cells, whole blood, serum, plasma, cerebrospinal fluid etc.

16. The method as claimed in claim 15, wherein said sample has been pretreated with hyaluronidase from *Streptomyces hyalurolyticus* (having no chondrotinase activity) and/or heparanase.

17. The method according to any of claims 13 to 16 for detecting a compound capable of modulating an endoglycosidase enzyme activity, comprising additionally the following step:
(iv) comparing the change in the amount of the bound substrate measured in the absence of the test compound with that measured in the presence of the test compound.

18. The method as claimed in any of claims 13 to 17, wherein the endoglycosidase is an enzyme of the hyaluroindase type selected from the family of vertebrate hyaluronidases, preferably from human hyaluronidases (Hyals 1-4, PH-20) and wherein the enzyme may be a recombinant hyaluronidase, purified hyaluronidase or non-purified hyaluronidase and preferably the enzyme is provided at least in a partially purified form.

19. The method as claimed in any of claims 13 to 18, wherein the substrate is selected from chondroitin sulfate proteoglycans, chondroitin sulfates or their respective derivatives.

20. The method as claimed in any of claims 13 to 19, wherein the chondroitin sulfate binding protein (CS-BP) is selected from protamine or other members of the family of arginine-rich protamine-like proteins from the histone family, and wherein these binding proteins were natural proteins, purified from sperm of different species, low molecular weight forms of these proteins, derived for example by proteolytic cleavage, peptides, or recombinant proteins.

21. The method as claimed in any of claims 13 to 20, wherein the substrate is covalently attached to a first member of a ligand/receptor pair and the second member is coupled to an enzyme as part of a detection system.

22. The method as claimed in claim 21, wherein the second member of the ligand/receptor pair is labelled with a fluorescent, chemiluminescent or electrochemiluminescent label.

23. The method as claimed in any of claims 13 to 20, wherein the substrate is covalently attached to a fluorescent, chemiluminescent or electrochemiluminescent compound and the fluorescence or luminescence signal of the substrate bound to the chondroitin sulfate binding protein (CS-BP) is measured directly.

24. A method for detecting a compound capable of modulating of the chondroitinase-like and the hyaluronidase-like enzyme activity of hyaluronidase as claimed in claim 17, wherein said test compound is selected from anti-hyaluronidase antibodies, natural products, synthetic products, products from a library of compounds obtained by combinatorial chemistry, peptides and proteins.

25. A kit preferably for carrying out a method as claimed in any of claims 13 to 24, comprising the following components:
(a) a substrate which can be cleaved by an enzyme having activity of the hyaluronidase type, in particular CS-(GAG) or peptido-CS (GAG) preferably labelled with a first member of a receptor/ligand pair,
(b) a vertebrate hyaluronidase, preferably one of the human hyaluronidases, which can be recombinant hyaluronidase, purified hyaluronidase or non-purified hyaluronidase,
(c) a detection system preferably comprising the second member of the receptor/ligand pair, coupled to an enzyme preferably avidin-peroxidase or streptavidin-peroxidase and a suitable enzyme substrate, and
(d) a CS-BP, preferably bound to a solid phase.

26. A kit to measure chondroitinase activity as claimed in claim 25 comprising:
- chondroitin sulfate (CS), preferably peptido-CS, labelled with biotin
- avidin or streptavidin coupled to peroxidase and a peroxidase substrate and
- a protamine-coated solid phase, preferably a microplate.

27. A kit to measure hyaluronidase activity as claimed in claim 25 comprising:
- components as in claim 26, and additionally hyaluronan.

28. A kit to measure the modulation of chondroitinase activity as claimed in claim 25 comprising the components of the kit according to claim 26 and additionally a vertebrate hyaluronidase (having chondroitinase activity).

29. A kit to measure the modulation of chondroitinase activity as claimed in claim 25 comprising the components of the kit according to claim 27 and additionally a vertebrate hyaluronidase (having chondroitinase activity).
